# EUROPÄISCHE PATENTSCHRIFT

(11) **EP 0 931 554 B1**
(45) Veröffentlichungstag und Bekanntmachung des Hinweises auf die Patenterteilung: **30.07.2008**
(21) Anmeldenummer: 99100911.9
(22) Anmeldetag: 20.01.1999
(51) Int. Cl.: A61M 1/36, A61M 1/02, B04B 5/04

(54) **Vorrichtung zur Aufbereitung von intra- oder postoperativen Blutverlusten für die Autotransfusion**
Device for the recovery of blood lost during and after surgery for autotransfusion
Dispositif de récupération des pertes de sang intra- et postopératoires pour autotransfusion

(30) Priorität: 23.01.1998 DE 19802321
(43) Veröffentlichungstag der Anmeldung: 28.07.1999
(73) Patentinhaber: Fresenius AG, 61352 Bad Homburg v.d.H. (DE)
(72) Erfinder: Biesel, Wolfgang, Dr., 66564 Ottweiler (DE)
(74) Vertreter: Luderschmidt, Schüler & Partner

(56) Entgegenhaltungen:
- EP-A- 0 155 684
- EP-A- 0 303 765
- DE-A- 4 226 974

## Beschreibung

Die Erfindung bezieht sich auf ein Verfahren zur Aufbereitung von intra- oder postoperativen Blutverlusten, bei welchem bestimmte Zeilen der aufzubereitenden Zellsuspension durch Zentrifugieren in einer Separationseinheit konzentriert und aus der Separationseinheit abgeführt werden. Darüber hinaus betrifft die Erfindung eine Vorrichtung zur Durchführung eines derartigen Verfahrens.

Aus dem Stand der Technik sind vielfältige Separationseinrichtungen und zugehörige Verfahren bekannt, bei welchen insbesondere Blut in seine Bestandteile getrennt und diese, beispielsweise Erythrozyten oder Plasma, einer weiteren Verwendung zugeführt werden.

Es gibt vielfältige medizinische Anwendungsfälle für derartige Verfahren und Vorrichtungen. Eines dieser Anwendungsgebiete ist die intraoperative Autotransfusion, die in der letzten Zeit breite Anwendung gefunden hat. Die intraoperative Autotransfusion ist ein Verfahren, das die Retransfusion von aus dem Operationsfeld gesammeltem Blut ermöglicht. Anwendung im Bereich der intraoperativen Autotransfusion finden sogenannte Vollblutretransfusionsverfahren, die das gesammelte Blut lediglich einer Partikelfiltration unterziehen, bis hin zu Plasmaseparations-/Waschverfahren, die ein gewaschenes Erythrozytenkonzentrat zur Reinfusion bereitstellen. Die Vorteile der Transfusion von autologen, d.h. patienteneigenem Blut liegen gegenüber der Transfusion von homologen Blut, d.h. Fremdblut, in der Vermeidung von Infektionskrankheiten, wie beispielsweise Aids, Hepatitis sowie in der Vermeidung von Transfusionsreaktionen aufgrund biologischer Inkompatibilität und Immunsystemreaktionen.

Im Rahmen der Entwicklung intraoperativer Autotransfusionstechniken hat es sich herausgestellt, daß die Transfusion von Vollblut gegenüber der Transfusion gewaschener Erythrozytenkonzentrate nachteilig sein kann. Diese Nachteile der Vollbluttransfusionsverfahren bestehen darin, daß unerwünschte Bestandteile des gesammelten Blutes nicht eliminiert werden können. Intraoperatives Blut enthält unbekannte Mengen an Hämolyseprodukten, aus dem Gewebe eingeschwemmten oder von außen zugeführten Fremdbestandteilen, überschüssiges Volumen, Antikoagulanzien, aktivierte plasmatische und zelluläre Gerinnungsfaktoren, Produkte der Gewinnungsaktivierung und des fibrinolytischen Systems. All diese Bestandteile können klinische Komplikationen auslösen, was zur Einschränkung des Einsatzgebietes geführt hat. Es ist aus dem Stand der Technik bekannt, bei derartigen Autotransfusionssystemen von Blut Filtersysteme einzusetzen, welche jedoch lediglich Blutgerinnsel oder Gewebeteile zurückhalten. Ein derartiges System ist aus der US-PS 4,014,329 bekannt. Die US-PS 4,886,487 beschreibt eine Vorrichtung zur Absonderung überschüssigem Fluids, wobei jedoch Gerinnungsfaktoren, Waschflüssigkeit, Antikoagulans und andere Additive mit dem Blut des Patienten rückgeführt werden.

Als Alternative zur Vollblutretransfusion wurden Plasmaseparations /Waschverfahren entwickelt, welche Zentrifugen verwenden. Derartige Zentrifugen sind in der DE-OS 22 62 856 und der WO 89/01792 beschrieben.

Die DE-PS 38 17 664 beschreibt eine Gegenstromextraktionszentrifuge, bei welcher Vollblut im Gegenstrom gegen eine Waschlösung geschleudert wird. Weder die Vorrichtung noch das Verfahren erfüllen jedoch die Anforderungen, welche im Rahmen einer Autotransfusion gestellt werden, da die unerwünschten Bestandteile nicht in sicherer und effektiver Weise abgetrennt werden können.

Die EP 0 303 765 beschreibt ein Verfahren zur Zellseparation, wobei dem Patienten das in seine Bestandteile zu zerlegende Blut mittels einer Kanüle entnommen und mit der gleichen Kanüle wieder zugeführt wird. Das bekannte Verfahren macht von einem Zellseparator Gebrauch, der über eine rotierende Membran verfügt. Das Blut des Patienten wird über eine an die Kanüle angeschlossene Zulaufleitung zu dem Zellseparator gefördert und über eine Rücklaufleitung von dem Zellseparator zurück zu der Kanüle gefördert. Das bekannte Verfahren zur Zellseparation ist nicht dazu bestimmt, intra- oder postoperative Blutverluste aufzubereiten. Vielmehr dient das bekannte Verfahren dazu, das dem Patienten mittels der Kanüle entnommene Blut, das nicht verunreinigt ist, in seine Bestandteile zu zerlegen.

Die EP 0 303 765 spricht das Problem an, dass für die ordnungsgemäße Funktion des Zellseparators erforderlich ist, ein bestimmtes Verhältnis zwischen dem Blutfluss in den Separator und dem Rückfluss zum Patienten einzuhalten. Daher schlägt die EP 0 303 765 zum einen vor, mit einer einzigen Pumpe sowohl den Zu- als auch Abfluss zu kontrollieren, und zum anderen eine vorgegebene Menge an Kochsalzlösung der aus dem Zellseparator strömenden Flüssigkeit hinzuzufügen.

Ein Verfahren zur Aufbereitung von Zellsuspensionen ist aus der EP 0 155 684 bekannt. Die konzentrierte Zelltraktion wird nach der Abführung aus dem Separator aber nicht mit einer physiologischen Lösung verdünnt. Die DE 42 26 974 C2 beschreibt ein Verfahren zur Aufbereitung von intraoperativen Blutverlusten, bei welchem die aufzubereitende Zellsuspension über eine Zuführungsleitung in eine Separationskammer geleitet und entsprechend der Dichte in ihre Bestandteile zerlegt wird. In einem zweiten Verfahrensschritt wird die konzentrierte Zellfraktion, vornehmlich Erythrozytenkonzentrat, durch kontinuierliche Zugabe einer Waschlösung resuspendiert. Anschließend werden die verbliebenen nicht-zellulären Bestandteile durch eine nochmalige Separation abgetrennt, wobei nach der Abführung der verunreinigten Waschlösung als Ergebnis ein hochreines Erythrozytenkonzentrat verbleibt. Zur Durchführung des Verfahrens findet eine Zentrifuge mit einer Separationskammer Verwendung, die einen Ringkanal aufweist. Der Ringkanal ist in drei Bereiche aufgeteilt, wobei in dem ersten Bereich die erste Separation der Zellsuspension, in dem zweiten Bereich die Resuspendierung und in dem dritten Bereich die zweite Separation der resuspendierten Zellen erfolgt.

Das obige Verfahren hat sich in der Praxis bewährt. Nachteilig ist jedoch, daß relativ große Volumina von Waschlösung zur Verdrängung bzw. Verdünnung des Restplasmas durch die Zentrifugenkammer geleitet werden müssen. Im Hinblick auf die großen Volumina wird als Waschlösung bei dem bekannten Verfahren im allgemeinen eine isotonische Kochsalzlösung verwendet. Der Einsatz der für den Patienten weitaus verträglicheren, aber teureren Ringerlösung anstelle der Kochsalzlösung ist wegen der großen Volumina mit relativ hohen Kosten verbunden.

Der Erfindung liegt die Aufgabe zugrunde, ein Verfahren zur Aufbereitung von intra- oder postoperativen Blutverlusten anzugeben, das die Gewinnung eines hochreinen Zellkonzentrates unter dem

Einsatz nur kleiner Volumina einer Verdünnungslösung erlaubt. Die Lösung dieser Aufgabe erfolgt mit den im Patentanspruch 1 angegebenen Merkmalen.

Eine weitere Aufgabe der Erfindung liegt darin, eine Vorrichtung zur Durchführung des Verfahrens zur Aufbereitung von intra- oder postoperativen Blutverlusten bereitzustellen. Diese Aufgabe wird mit den Merkmalen des Patentanspruchs 5 gelöst.

Das erfindungsgemäße Verfahren zur Aufbereitung einer Zellsuspension, insbesondere zur kontinuierlichen Aufbereitung von intra- oder postoperativen Blutverlusten zu einem Erythrozytenkonzentrat, beruht darauf, daß die Aufbereitung ohne erneutes Aufspülen oder Waschen der konzentrierten Zellen durchgeführt wird. Bei dem erfindungsgemäßen Verfahren werden bestimmte Zellen der aufzubereitenden Zellsuspension, vornehmlich die Erythrozyten, durch Zentrifugieren in einer Separationseinheit konzentriert und aus der Separationseinheit abgeführt, ohne daß die konzentrierten Zellen in der Separationseinheit aufgespült oder gewaschen werden. Überraschenderweise hat sich gezeigt, daß die Separation nicht nur weitaus schneller, sondern auch insofern effektiver ist, als das gewonnene Zellkonzentrat, insbesondere das Erythrozytenkonzentrat, eine höhere Reinheit hat, d.h. geringere Kontamination mit Leukozyten.

Zur Verdünnung des gewonnenen Zellkonzentrats sind nur geringe Volumina einer Verdünnungslösung erforderlich. Daher ist der Einsatz der für den Patienten weitaus verträglicheren Ringerlösung anstelle der Kochsalzlösung nicht mit wesentlich höheren Kosten verbunden. Anstelle einer Ringerlösung kann das Zellkonzentrat aber auch mit Lagerlösungen für Blutzellen u.ä. verdünnt werden.

Von Vorteil ist auch, daß sich der gewünschte Hämatokrit des Endproduktes durch Wahl des Verdünnungsverhältnisses frei einstellen läßt. Auch werden große, mit Blutbestandteilen kontaminierte Abfallvolumina vermieden. Da Zellverluste in einem Waschprozeß nicht auftreten können, ist die Gewinnung von Erythrozyten sehr effizient. Die plasmatischen Bestandteile des Blutes können unverdünnt zur weiteren Verwendung gesammelt werden.

Die Vorrichtung zur Durchführung des erfindungsgemäßen Verfahrens hat insofern einen vereinfachten Aufbau, als an der Separationseinheit eine separate Zulaufleitung für eine Waschlösung nicht erforderlich ist. Damit kann die Separationseinheit, die vorzugsweise als Disposable ausgebildet ist, kostengünstig hergestellt werden.

Die Verdünnung des Zellkonzentrats erfolgt vorzugsweise unmittelbar nach der Abführung aus der Separationseinheit. Bei einer bevorzugten Ausführungsform der erfindungsgemäßen Vorrichtung verfügt die Einrichtung zur Verdünnung der aus der Separationseinheit abgeführten Zellfraktion ein Behältnis zur Aufnahme der physiologischen Verdünnungslösung und eine von dem Behältnis abgehende und in die Ablaufleitung für die konzentrierte Zellfraktion mündende Zulaufleitung. Der Mischpunkt, an dem die Zulaufleitung für die Verdünnungslösung in die Ablaufleitung für das Zellkonzentrat mündet, liegt dabei vorzugsweise stromauf der Förderpumpe, mit der die Zellfraktion aus der Separationseinheit abgezogen wird. Dadurch werden Hämolyseeffekte bedingt durch hohe Zellkonzentration und Scherkräfte minimiert.

Um den Hämatokrit des Endproduktes frei vorgeben zu können, verfügt die Verdünnungseinrichtung über eine Steuereinheit, mit der die Fördermenge der Pumpe zum Zuführen der Verdünnungslösung eingestellt werden kann.

Im folgenden wird die Erfindung anhand eines Ausführungsbeispiels unter Bezugnahme auf die Zeichnungen näher erläutert.

Es zeigen:
- Figur 1: ein Ausführungsbeispiel der erfindungsgemäßen Vorrichtung zur Aufbereitung von intra- oder postoperativen Blutverlusten für die Autotransfusion in vereinfachter schematischer Darstellung und
- Figur 2: die Separationseinheit der Vorrichtung zur Durchführung des Verfahrens von Figur 1 in vereinfachter schematischer Darstellung.

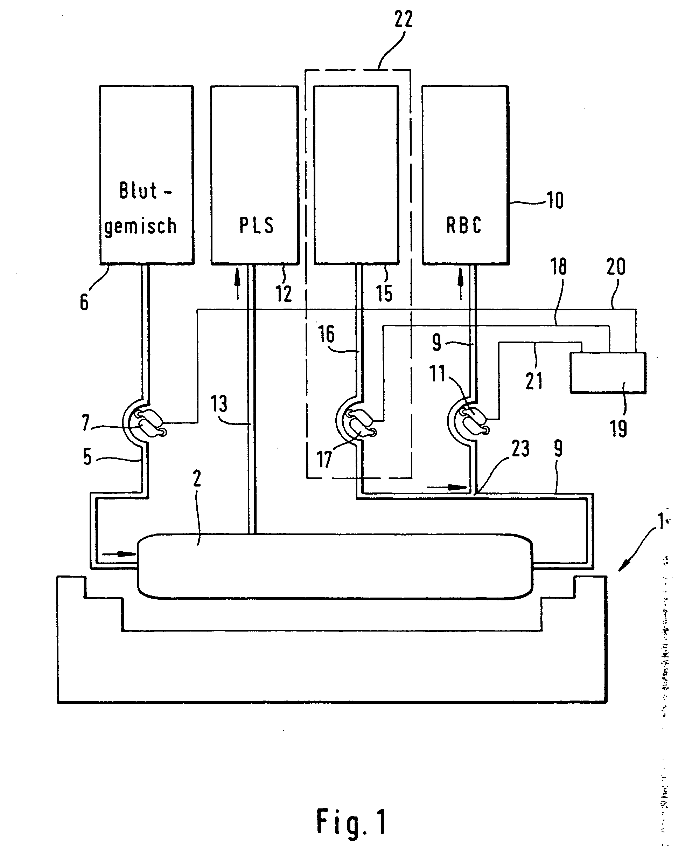

Die Vorrichtung umfaßt eine Zentrifuge 1 mit einer als Disposable ausgebildeten und in die Zentrifuge eingesetzten Zentrifugenkammer 2. Diese Bauteile sind in Figur 1 nur andeutungsweise dargestellt.

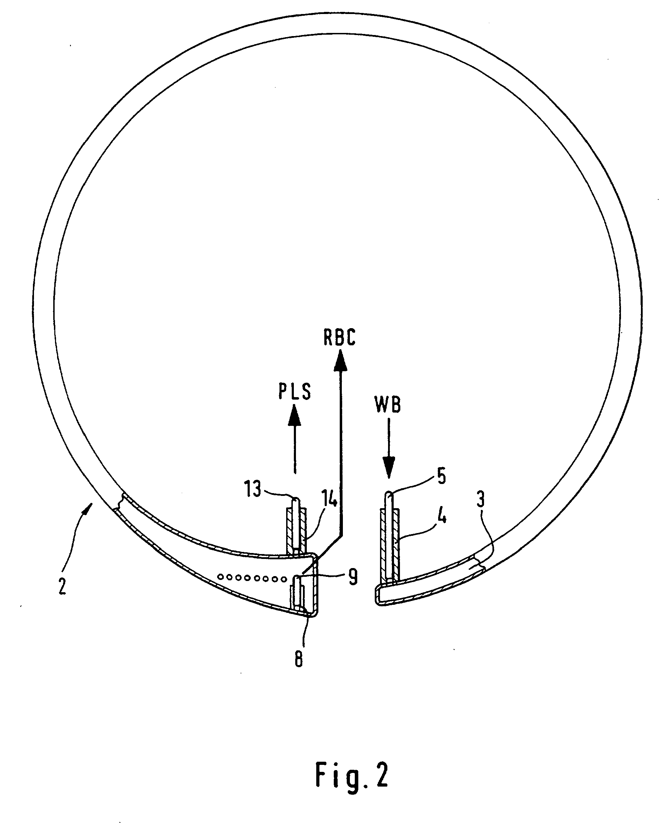

Die Zentrifugenkammer 2 weist einen Ringkanal 3 auf (Figur 2). An der Innenseite des schmalen Ringkanalendes ist ein Anschluß 4 vorgesehen, an dem eine Zulaufleitung 5 zum Zuführen der aufzubereitenden Zellsuspension angeschlossen ist, die von einem Behältnis 6 abgeht, das während oder nach einer Operation gesammeltes Blutgemisch eines Patienten enthält. In die Zulaufleitung 5 ist eine okkludierende Blutpumpe 7 geschaltet.

An der Außenseite des Ringkanalendes ist ein Anschluß 8 vorgesehen, an den eine erste Ablaufleitung 9 für die konzentrierte Zellfraktion, d.h. das Erythrozytenkonzentrat (RBC), angeschlossen ist, die zu einem Sammelbehältnis 10 führt. In die erste Ablaufleitung 9 ist eine okkludierende Konzentratpumpe 11 geschaltet.

Eine zu einem weiteren Sammelbehältnis 12 führende zweite Ablaufleitung 13 für nicht benötigte Bestandteile (Plasma, PLS) ist an einem Anschluß 14 der Separationskammer angeschlossen, der an der Innenseite des Ringkanal 3 vorgesehen ist.

Während des Betriebs der Vorrichtung wird das aufzubereitende Blutgemisch mittels der Blutpumpe 7 in die rotierende Separationskammer 2 gefördert, in deren Ringkanal 3 sich das Vollblut unter dem Einfluß der Zentrifugalkraft aufgetrennt, wobei sich die Erythrozyten an der Außenseite des Ringkanals absetzen und mittels der Konzentratpumpe 11 aus der Separationskammer über die erste Ablaufleitung 9 kontinuierlich abgezogen und in dem Behältnis 10 gesammelt werden. Demgegenüber werden die nicht benötigten Bestandteile (Gewebewasser/Plasma) des Blutgemisches kontinuierlich über die zweite Ablaufleitung 13 abgeführt und in dem Behältnis 12 gesammelt.

Die erfindungsgemäße Vorrichtung verfügt des weiteren über eine Einrichtung 22 zur Verdünnung des über die erste Ablaufleitung 9 abgezogenen Erythrozytenkonzentrats. Die Verdünnungseinrichtung 22 umfaßt ein Behältnis 15 zur Aufnahme der Verdünnungslösung, vorzugsweise eine Ringerlösung, von dem eine Zulaufleitung 16 abgeht, die an einem Mischpunkt 23 stromauf der Konzentratpumpe 11 in die erste Ablaufleitung 9 mündet. In die Zulaufleitung 16 ist eine okkludierende Förderpumpe 17 geschaltet, die über eine Steuerleitung 18 an einer zentralen Steuereinheit 19 angeschlossen ist. Die Steuereinheit 19 ist über weitere Steuerleitungen 20, 21 auch mit der Blutpumpe 7 und der Konzentratpumpe 11 verbunden. Mit der Steuereinheit können die Fördermengen der Pumpen 7, 11, 17 eingestellt werden. Durch entsprechende Einstellung der Fördermenge der Pumpe 17 bzw. der Pumpe 11 kann der gewünschte Hämatokrit des dem Patienten rückzuführenden Erythrozytenkonzentrats vorgegeben werden.

In der Separationskammer 2, die ring- oder spiralförmig ausgebildet sein kann, erfolgt eine Separation des Blutgemisches auf einen Hämatokrit von 60 bis 98, vorzugsweise 85.

Der nachfolgenden Tabelle sind die mit dem erfindungsgemäßen Verfahren erzielbaren Plasma- und Leukozyteneliminationsraten bei einem Hämatokrit des Blutes von 20% und einem Hämatokrit des Erythrozytenkonzentrates von 70, 80 und 90% zu entnehmen.

| **Hkt (Blut) [%]** | **Hkt (EK) [%]** | **Plasma-Eliminationsrate [%]** | **Leukozyten-Eliminationsrate [%]** |
|---|---|---|---|
| 20 | 70 | 89 | ca. 80 |
| 20 | 80 | 94 | ca. 90 |
| 20 | 90 | 97 | > 90 |

## Patentansprüche

1. Verfahren zur Aufbereitung von intra- oder postoperativen Blutverlusten, bei dem während oder nach einer Operation gewonnene intra- oder postoperative Blutverluste in einem Behältnis gesammelt werden und bestimmte Zellen der aufzubereitenden Zellsuspension durch Zentrifugieren in einer Separationseinheit konzentriert und aus der Separationseinheit abgeführt werden, **dadurch gekennzeichnet, dass** die konzentrierte Zellfraktion nach der Abführung aus der Separationseinheit mit einer physiologischen Lösung verdünnt wird.

2. Verfahren nach Anspruch 1, **dadurch gekennzeichnet, dass** die konzentrierte Zellfraktion ein Erythrozytenkonzentrat ist.

3. Verfahren nach Anspruch 2, **dadurch gekennzeichnet, dass** die Konzentrierung der Zellen auf Hämatokritwerte von 60 bis 98 erfolgt.

4. Verfahren nach einem der Ansprüche 1 bis 3, **dadurch gekennzeichnet, dass** als physiologische Lösung eine Ringerlösung verwendet wird.

5. Vorrichtung zur Durchführung des Verfahrens nach einem der Ansprüche 1 bis 4, mit einem Behältnis (6) zum Sammeln von intra- oder postoperativen Blutverlusten, einer Zentrifuge (1), welche zumindest eine Separationseinheit (2) mit einem Ringkanal (3) aufweist, an den eine von dem Behältnis (6) zum Sammeln der intra- oder postoperativen Blutverluste abgehende Zulaufleitung (5) für die aufzubereitende Zellsuspension, eine erste Ablaufleitung (9) für die konzentrierte Zellfraktion und eine zweite Ablaufleitung (13) für die nicht benötigten Bestandteile der Zellsuspension angeschlossen sind, **dadurch gekennzeichnet, dass** eine Einrichtung (22) zur Verdünnung der über die erste Ablaufleitung (9) aus der Separationseinheit (2) abgeführten Zellfraktion vorgesehen ist.

6. Vorrichtung nach Anspruch 5, **dadurch gekennzeichnet, dass** die Verdünnungseinrichtung (22) ein Behältnis (15) zur Aufnahme einer physiologischen Verdünnungslösung und eine von dem Behältnis abgehende und in die erste Ablaufleitung (9) für die konzentrierte Zellfraktion mündende Zulaufleitung (16) aufweist, in die eine Förderpumpe (17) zum Zuführen der Verdünnungslösung geschaltet ist.

7. Vorrichtung nach Anspruch 6, **dadurch gekennzeichnet, dass** in die erste Ablaufleitung (9) für die konzentrierte Zellfraktion eine Konzentratpumpe (11) zum Abziehen der Zellfraktion aus der Separationseinheit (2) geschaltet ist, wobei der Mischpunkt (23), an dem die Zulaufleitung (16) für die Verdünnungslösung in die erste Ablaufleitung (9) für das Zellkonzentrat mündet, stromauf der Konzentratpumpe (11) liegt.

8. Vorrichtung nach Anspruch 6 oder 7, **dadurch gekennzeichnet, dass** die Verdünnungseinrichtung (22) eine Steuereinheit (19) zur Einstellung der Fördermenge der Pumpe (17) zum Zuführen der Verdünnungslösung aufweist.

## Claims

1. A method for processing intra-operative or post-operative blood losses, wherein intra-operative or post-operative blood losses obtained during or after an operation are collected in a container and certain cells of the cell suspension to be processed are concentrated by centrifugation in a separation unit and conveyed away from the separation unit, **characterised in that** the concentrated cell fraction, after having been conveyed away from the separation unit, is diluted with a physiological solution.

2. The method according to claim 1, **characterised in that** the concentrated cell fraction is an erythrocyte concentrate.

3. A method according to claim 2, **characterised in that** the concentration of the cells takes place to haematocrit values of 60 to 98.

4. The method according to any one of claims 1 to 3, **characterised in that** a Ringer's solution is used as the physiological solution.

5. A device for performing the method according to any one claims 1 to 4, with a container (6) for collecting intra-operative or post-operative blood losses, a centrifuge (1), which comprises at least one separation unit (2) with an annular channel (3), to which there are connected a supply line (5) for the cell suspension to be processed, said supply line leading away from the container (6) for collecting the intra-operative or post-operative blood losses, a first discharge line (9) for the concentrated cell fraction and a second discharge line (13) for the constituents of the cell suspension that are not required, **characterised in that** a device (22) is provided for diluting the cell fraction conveyed away via the first discharge line (9) from the separation unit (2).

6. The device according to claim 5, **characterised in that** the dilution device (22) comprises a container (15) for accommodating a physiological dilution solution and a supply line (16), which departs from the container and emerges into the first discharge line (9) for the concentrated cell fraction and into which a feed pump (17) for supplying the dilution solution is incorporated.

7. The device according to claim 6, **characterised in that** a concentrate pump (11) for drawing off the cell fraction from the separation unit (2) is incorporated into the first discharge line (9) for the concentrated cell fraction, the mixing point (23) at which the supply line (16) for the dilution solution emerges into the first discharge line (9) for the cell concentrate lying upstream of the concentrate pump (11).

8. The device according to claim 6 or 7, **characterised in that** the dilution device (22) comprises a control unit (19) for adjusting the delivery quantity of the pump (17) for supplying the dilution solution.

## Revendications

1. Procédé de traitement du sang perdu pendant ou après une opération, dans lequel les pertes sanguines intra-opératoires ou postopératoires, récupérées pendant ou après une opération, sont collectées dans un récipient et une partie des cellules de la suspension cellulaire à traiter est concentrée par centrifugation dans une unité de séparation et est évacuée hors de l'unité de séparation, **caractérisé en ce que** la fraction cellulaire concentrée issue de l'évacuation hors de l'unité de séparation, est diluée avec une solution physiologique.

2. Procédé selon la revendication 1, **caractérisé en ce que** la fraction cellulaire concentrée est un concentré d'hérythrocytes.

3. Procédé selon la revendication 2, **caractérisé en ce que** la concentration des cellules permet d'obtenir des valeurs d'hématocrite de 60 à 98.

4. Procédé selon l'une quelconque des revendications 1 à 3, **caractérisé en ce que** la solution physiologique est une solution de Ringer.

5. Dispositif destiné à la mise en oeuvre du procédé selon l'une quelconque des revendications 1 à 4, comportant un récipient (6) destiné à collecter le sang perdu pendant ou après une opération, une centrifugeuse (1), qui comporte au moins une unité de séparation (2) avec un canal annulaire (3), auquel sont raccordées un conduit d'admission (5) pour la suspension cellulaire à traiter, partant du récipient (6) destiné à collecter le sang perdu pendant ou après une opération, un premier conduit d'évacuation (9) de la fraction cellulaire concentrée et un deuxième conduit d'évacuation (13) des composants non nécessaires de la suspension cellulaire, **caractérisé en ce qu'**il est prévu un dispositif (22) pour la dilution de la fraction cellulaire évacuée hors de l'unité de séparation (2) via le premier conduit d'évacuation (9).

6. Dispositif selon la revendication 5, **caractérisé en ce que** le dispositif de dilution (22) comporte un récipient (15) destiné à recevoir une solution de dilution physiologique, et un conduit d'admission (16), qui débouche dans le premier conduit d'évacuation (9) de la fraction cellulaire concentrée et sur lequel est fixée une pompe de refoulement (17) pour acheminer la solution de dilution.

7. Dispositif selon la revendication 6, **caractérisé en ce que** sur le premier conduit d'évacuation (9) de la fraction cellulaire concentrée est fixée une pompe de concentration (11) pour aspirer la fraction cellulaire hors de l'unité de séparation (2), **caractérisé en ce que** le point de mélange (23), au niveau duquel le conduit d'admission (16) de la solution de dilution débouche dans le premier conduit d'évacuation (9) du concentré cellulaire, est situé en amont de la pompe de concentration (11).

8. Dispositif selon la revendication 6 ou 7, **caractérisé en ce que** le dispositif de dilution (22) comporte une unité de commande (19) pour régler le débit de la pompe (17) destinée à acheminer la solution de dilution.
